Europäisches Patentamt

European Patent Office

Office européen des brevets

(19)

(11) Publication number: **0 274 411**
**A2**

## (12) EUROPEAN PATENT APPLICATION

(21) Application number: 88300025.9

(51) Int. Cl.⁴: **A 61 M 29/02**

(22) Date of filing: 05.01.88

(30) Priority: 09.01.87 US 1759

(43) Date of publication of application:
13.07.88 Bulletin 88/28

(84) Designated Contracting States:
BE CH DE ES FR GB IT LI NL

(71) Applicant: C.R. Bard, Inc.
731 Central Avenue
Murray Hill, New Jersey 07974 (US)

(72) Inventor: Saab, Mark Anthony
16 Nesmith Street
Lawrence Massachusetts 01841 (US)

(74) Representative: Woodward, John Calvin et al
VENNER SHIPLEY & CO. 368 City Road
London EC1V 2QA (GB)

(54) Thin wall high strength balloon and method of manufacture.

(57) A balloon for use in medical procedures such as in dilatation catheter in which the balloon is formed from a tubular thin wall parison of orientable semicrystalline polymer (polyethylene terephthalate being preferred) which is stretched very close to the elastic limit of the material and is then heat set. The balloon thus produced is extremely strong and has a very thin and higly flexible wall and is dimensionally stable both in storage and when inflated. The preferred balloon has a wall thickness to diameter ratio of less than 5.0 x 10-3 and a radial tensile strength (hoop strength) greater than 35,000 psi.

EP 0 274 411 A2

**Description**

THIN WALL HIGH STRENGTH BALLOON AND METHOD OF MANUFACTURE

FIELD OF THE INVENTION

This invention relates to improvements in balloons used in medical procedures such as in dilatation catheters.

BACKGROUND OF THE INVENTION

This invention concerns balloon dilatation procedures in which a catheter carrying a balloon on its distal end is placed within a body cavity of a patient and is inflated to dilate the cavity. The procedure is commonly employed to dilate a stenosed artery, and is often performed to dilate obstructed coronary arteries. U.S. Patent 4,195,637 to Gruntzig discloses one type of dilatation catheter having a balloon at its distal end and adapted to perform a dilatation procedure. The balloon is intended to have a maximum inflated diameter. Typically, the physician will select a catheter having a balloon with an inflated diameter corresponding to the inner diameter of the unobstructed blood vessel adjacent the stenosis to be treated. Dilatation procedures also are performed in other portions of the body, as in valvuloplasty, in which a dilatation balloon is placed and is inflated within a patient's heart valve to dilatate the commissures which have become immobile due to calcification, adhesion or other causes and to permit the valve to function more effectively.

Dilatation balloons have been made from various materials including polyvinyl chloride, polyethylene and, most recently, polyethylene terephthalate, as described in U.S. Patent 4,490,241 to Levy.

It is desirable for a dilatation balloon to have several features. The balloon should have a thin wall so that it can fold down closely about the catheter shaft to a low profile, thereby enabling the deflated balloon to be inserted into and removed through narrow stenoses and passageways. In heart valve dilatations, it is important that the large dilatation balloon be capable of folding down closely to the catheter shaft in a low profile, to facilitate percutaneous entry of the catheter into relatively small diameter arteries and passage of the catheter through those arteries, into the aorta and toward the heart valve to be treated as well as when removing the catheter. The balloon also should not be stiff, as stiffness detracts from the ability of the balloon portion of the catheter to bend as it is advanced through tortuous passages, a characteristic sometimes referred to as "trackability". Low stiffness (high flexibility) also is important to enable the balloon to be folded easily within the patient's body when the balloon is deflated and advanced or withdrawn. In this regard, it should be understood that the balloon when deflated, typically tends to collapse to form a pair of wings which, if not sufficiently flexible, will not fold or wrap easily about the catheter body as the deflated balloon catheter is advanced or withdrawn against body tissue. The balloon also should have a sufficiently high burst strength to enable it to impart sufficient dilatation force to the vessel to be treated. However, because different procedures and different size vessels require balloons of different diameters, the burst strength for the different balloons required for the different procedures may vary considerably. This results from the fact that the dilating force of a dilatation balloon increases as a function of the diameter of the balloon, without requiring a corresponding increase in the inflation pressure. Thus, the larger the diameter of the balloon, the lower its burst strength may be while still developing sufficient dilatation force. For example, a 20 mm diameter balloon used in valvuloplasty procedure need only have a burst strength of about 3 to 6 atm whereas a 3 mm balloon used in the dilatation of small coronary arteries may require a burst pressure of 10 to 20 atm. Larger diameter balloons do not require as much internal pressure to create the same dilating force, therefore, there are many dilatation balloons in which high burst strength is not advantageous.

Another desirable feature of dilatation balloons is that they should be dimensionally stable, that is, they should retain their size and structural integrity during storage and, when inflated, should not exhibit excessive radial expansion beyond the nominal inflated diameter.

The dilatation balloons of the prior art have been deficient in one or more of the foregoing characteristics. For example, Levy Patent 4,490,421 relates to a balloon intended to have a high burst pressure with low radial expansion when placed under pressure. Balloons made according to the Levy '421 patent, however, are neither sufficiently thin walled nor flexible, and are not sufficiently dimensionally stable during inflation or when stored. The balloons may shrink during storage and, consequently, will not inflate to the nominal diameter expected by the physician and required for the particular procedure.

It is among the general objects of the invention to provide improved dilatation balloons and a method for their manufacture which provides superior properties of thin walls, flexibility and high strength as well as full dimensional stability both in storage and when inflated.

SUMMARY OF THE INVENTION

In accordance with the invention, the balloon is formed from a tubular thin wall parison of orientable semicrystalline polymer (polyethylene terephthalate being preferred) which is stretched very close to the elastic limit of the material and is then heat set. The balloon thus produced is extremely strong and has a very thin and highly flexible wall and is dimensionally stable both in storage and when inflated. Thus, it is among the objects of the invention to provide dilatation balloons having superior properties of thin walls, high tensile strength and dimensional stability. More specifically, it is among the objects of the invention to provide dilatation balloons having radial tensile strength (hoop strength) greater than 35,000 psi. It also is among the objects of the invention to provide dilatation balloons and dilatation catheters having low profiles with superior trackability and balloon foldability. Also among the objects of the invention is to provide a method for making such balloons. The invention thus provides for a method for making a family of such balloons which display the foregoing properties and which are usable in a variety of medical procedures.

## DESCRIPTION OF THE DRAWINGS

The foregoing and other objects and advantages of the invention will be appreciated from the following further description with reference to the accompanying drawings wherein:

FIG. 1 is an illustration, in section, of a multi-part jacketed mold having the balloon formed within the mold and illustrating the tubular parison in phantom; and

FIG. 2 shows the difference in dimensional stability between the balloon made in accordance with the invention in which the balloon is heat set and a balloon which omits heat setting.

## DESCRIPTION OF THE PREFERRED EMBODIMENT

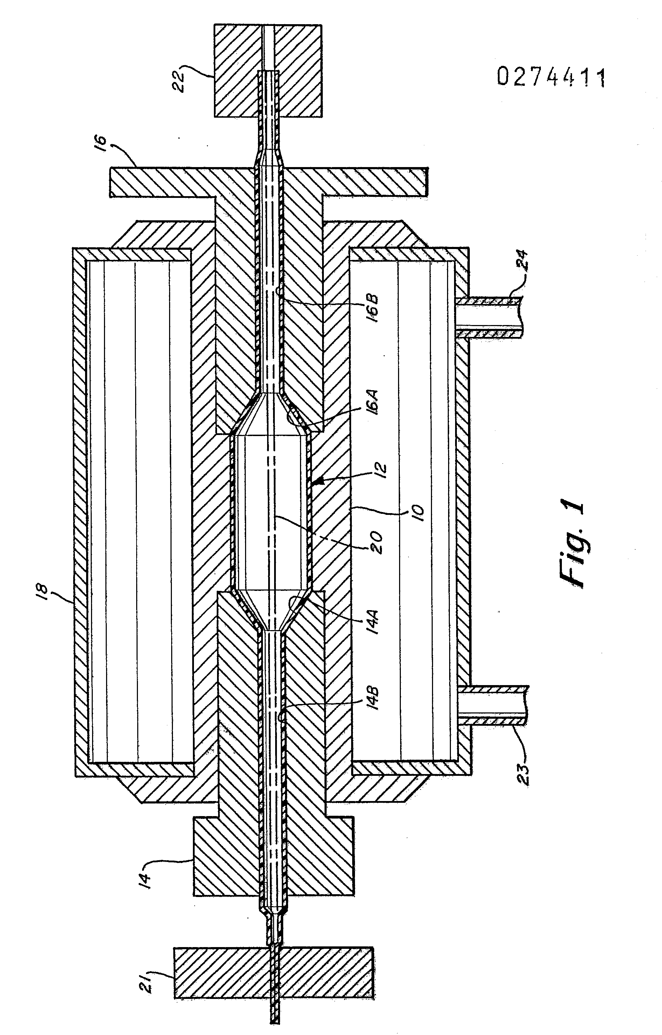

The balloon may be formed in a mold as illustrated in FIG. 1 which includes a mold body 10 having an internal bore which defines the intended dimension of the finished balloon, indicated at 12, and a pair of end members including a fixed end member 14 and a movable end member 16. Both end members include outwardly tapering portions 14A, 16A, respectively, which merge into smaller diameter end bores 14B, 16B, respectively. A water jacket 18 having inlet and outlet ports 23, 24 surrounds the mold 10. The mold parts are formed from a material, such as brass having good heat conductivity.

The mold receives a tubular parison, indicated in phantom at 20 in FIG. 1. The parison 20 is gripped at its ends which extend outwardly of the mold, one of the ends being sealed and the other end being connected securely to a source of fluid (such as a gas) under pressure as by a fitting 22. The clamp 21 and fitting 22 are mounted, by means not shown, to enable them to be drawn apart axially so as to impart an axial stretch to the parison 20.

The parison is formed from an orientable semicrystalline polymer such as polyethylene terephthalate (PET). In accordance with the invention, the parison is dimensioned with respect to the intended final configuration of the balloon. It is particularly important that the parison is relatively thin walled and that it is highly oriented being stretched radially close to the elastic limit of the material at the inner surface of the tube. The wall thickness is considered relative to the inside diameter of the parison tube. The present invention relates to thin walled parisons which have wall thickness-to-inside diameter ratios of less than 0.5 and, preferably between 0.45 and 0.09 or even lower. The use of such a thin walled parison enables the parison to be stretched radially to a greater and more uniform degree because there is less stress gradient through the wall from the surface of the inside diameter to the surface of the outside diameter. By utilizing thin wall starting parisons, there is less difference in the degree to which the inner and outer surfaces of the tubular parison are stretched. By maintaining a lowered stretch gradient across the wall of the parison, it is possible to produce a more uniformly and more highly oriented balloon having tensile strengths substantially higher than those of the prior art.

Orientation takes place at an elevated temperature, as controlled by a heat transfer fluid, such as hot water, circulated through the water jacket. The PET parison preferably is drawn axially and while being so drawn, is expanded radially within the mold. The orientation takes place at a temperature between the first and second order transition temperatures of the material, preferably about $80°C-99°C$ and more preferably at about $90°C$. The tube is drawn from a starting length L1 to a drawn length L2 which preferably is between 2.5 to 6 L1. The tubular parison, which was an initial internal diameter ID1 and outer diameter OD1 is expanded by gas emitted under pressure to the parison through fitting 22 to an internal diameter $ID_2$ which preferably is 6 to 8 ID1 and an outer diameter $OD_2$ which is about equal to or preferably greater than 4 $OD_1$. The expanded balloon then is subjected to a heat set step in which steam is circulated through the jacket 18 at a temperature above the stretching temperature and between $110°C$ to $220°C$ and preferably about $130°-170°C$ and is maintained for a fraction of a second or more, and preferably between about 5 to 30 seconds sufficiently to increase the degree of crystallinity in the balloon. The heat setting step is significantly in assuring dimensional stability for the balloon, both during storage and also under inflation pressures. After the heat set step, the mold is cooled to a temperature less than the second order transition temperature of the material. The balloon thus formed may be removed from the mold by removing the end piece 16 and withdrawing the formed balloon from the mold.

The degree of radial stretch in the resulting balloon is very close to the elastic limit of the material (at the inner surface) and, preferably is within 10% of the maximum achievable radial expansion for the material under the process conditions used. The high degree of orientation strengthens the balloon to tensile strengths of more than 35,000 psi and as high as 90,000 psi or more. This is to be compared with the prior art PET balloons described in the Levy '421 patent in which the highest tensile strengths achieved were approximately 34,000 psi. The resulting balloons are characterized by a very high ultimate tensile strength, a very thin and highly flexible balloon wall and dimensional stability in storage as well as under inflation conditions. For example, balloons made in accordance with the invention have a wall thickness to balloon diameter ratio (T/D) of the order of $5 \times 10^{-3}$ to $8 \times 10^{-5}$, with tensile strengths as high as 90,000 psi.

Table 1 below illustrates the T/D ratios for finished balloons of the present invention in comparison with T/D ratios for prior art balloons. Data for the balloons identified A-E were taken from Levy patent 4,490,421 for the correspondingly designated balloons in that patent.

## TABLE 1

### WALL THICKNESS (T)/BALLOON DIAMETER (D) RATIO

#### PRIOR ART

|  | A | B | C | D | E |
|---|---|---|---|---|---|
| T | .028 mm | .038 mm | .028 mm | .038 mm | .045 mm |
| D | 3.7 mm | 5.0 mm | 3.7 mm | 5.0 mm | 6.0 mm |
| T/D | $7.57 \times 10^{-3}$ | $7.6 \times 10^{-3}$ | $7.57 \times 10^{-3}$ | $7.6 \times 10^{-3}$ | $7.5 \times 10^{-3}$ |

#### PRESENT INVENTION

|  | F | G | H | I |
|---|---|---|---|---|
| T | .0136 mm | .0054 mm | .00806 mm | .0016 mm |
| D | 3.0 mm | 3.0 mm | 20.0 mm | 20.0 mm |
| T/D | $4.53 \times 10^{-3}$ | $1.8 \times 10^{-3}$ | $4.03 \times 10^{-4}$ | $8.05 \times 10^{-5}$ |

The balloons of the present invention display a remarkable increase in flexibility as compared to the prior PET balloons (Levy '421 patent). Flexibility is a function of the cube of the wall thickness, assuming all other variables, such as balloon material, balloon diameter, etc. are held constant. Table 2 illustrates the effect on flexibility of the thinner balloons of the present invention as compared with those of the prior art.

## TABLE 2

| | Prior art (Levy) | | | Saab Disclosure | | | Improvement in |
|---|---|---|---|---|---|---|---|
| Burst Pressure (Bars) | Radial Tensile Strength (psi) | Wall Thickness (mils) | Relative Stiffness $(t^3)$ | Radial Tensile Strength (psi) | Wall Thickness (mils) | Relative Stiffness $(t^3)$ | Flexibility Over Prior Art $\frac{t^3 - T^3}{T^3}$ (%) |
| 5 | 34,000 | 0.13 | 0.0022 | 90,000 | 0.048 | 0.00011 | 1,900 |
| 10 | 34,000 | 0.25 | 0.016 | 75,000 | 0.114 | 0.0015 | 1,000 |
| 15 | 34,000 | 0.38 | 0.054 | 65,000 | 0.20 | 0.0077 | 600 |
| 20 | 34,000 | 0.50 | 0.127 | 52,000 | 0.33 | 0.036 | 250 |
| 25 | 34,000 | 0.63 | 0.249 | 40,000 | 0.53 | 0.15 | 65 |

From Table 2 it can be seen that for balloons having burst pressures up to about 25 bars, the relative stiffness of the balloons of the present invention is far less than that of the prior art PET balloons. The flexibility of the balloons of the present invention, considered as a function of the cube of the thickness ($t^3$) of the balloons far exceeds those of the prior art. In the data presented in Table 2, the radial tensile strength was calculated at burst pressure. Wall thicknesses were calculated using the well known pressure vessel equation

$$S_c = \frac{PD}{2t}$$

where $S_c$ is the radial tensile strength, P is the burst pressure and D is the diameter of the balloon and t is the wall thickness. Wall thicknesses similarly where calculated and the thicknesses were cubed to provide an indication of relative stiffness.

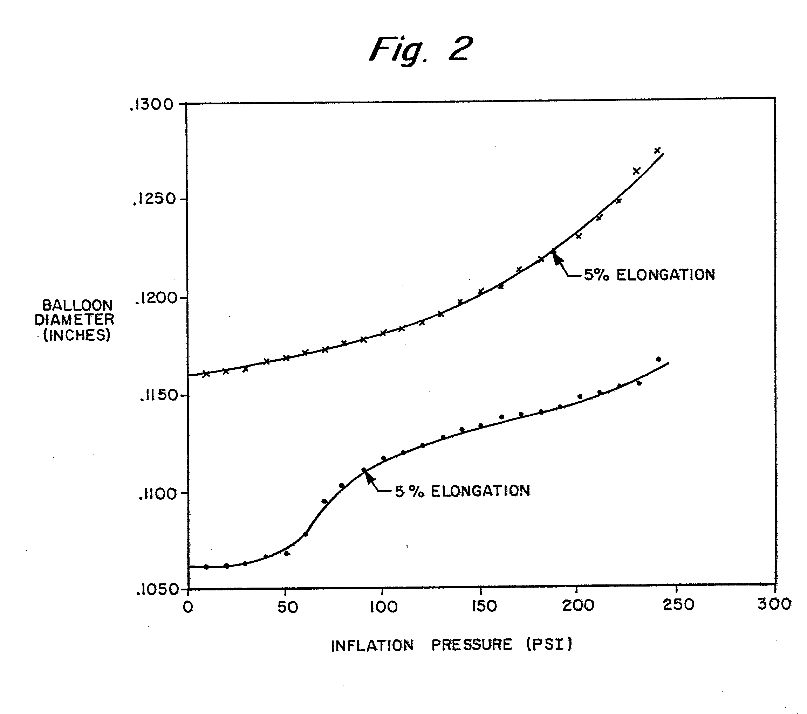

FIG. 2 shows the effect of the heat set step in the practice of the invention and compares the dimensional stability in storage as well as when inflated of a balloon which has been heat set (curve A) to one which has not been heat set (curve B). Both balloons were formed in a cylindrical mold having a diameter of 0.117", under identical conditions of temperature, time and pressure except that the heat set balloon was subjected to the heat set procedure as described above. The other balloon was cooled following the radial expansion, without any heat set. The balloons were removed from their molds and were permitted to remain in ambient conditions (20°C) for 48 hours. The balloons then were tested by inflating them under increasing pressures and measuring the balloon diameter as the pressure was increased. As shown in FIG. 2, the heat set balloon, when nominally inflated to remove wrinkles (about 10 psi) had a diameter of about 0.116", reflecting a shrinkage of 0.001". By contrast, the non-heat sealed balloon showed a nominal diameter of 0.1060, indicating a very substantial diametral shrinkage of 0.011". As the pressure was increased, the heat set balloon showed relatively steady and slow yielding, reaching 5% radial expansion at about 170 psi. In contrast, the non-heat set balloon displayed very substantially yielding and radial expansion in the range of 50 to 100 psi, reaching 5% radial expansion at about 85 psi.

The superior properties of balloons made in accordance with the invention may be appreciated further from the "F5" characteristics of the balloon. In oriented polymers the tensile strength at 5% elongation is referred to as "F5". FIG. 2 illustrates, on curve A, the point on which a balloon made in accordance with the invention has achieved 5% radial expansion. In curve A the F5 point occurs at about 170 psi inflation pressure. The balloon reflected in curve A is the balloon identified as balloon G in Table 1 (and in Example 2 below), having a wall thickness of 0.0054 mm and a diameter of 3.0 mm. From the pressure vessel equation, the radial tensile strength at 5% radial expansion is calculated to be 47,600 psi. That is contrasted with the prior art (Levy '421) balloons in which the maximum tensile strength achievable to burst is of the order of 34,000 psi. Thus, the present invention provides for F5 radial tensile strengths greater than 30,000 psi.

The following examples illustrate several balloons which may be made in the range of balloons achievable in accordance with the present invention.

## Example 1

A tubular parison was extruded from high molecular weight PET homopolyester resin having an initial intrinsic viscosity of about 1.04. The parison had an inner diameter of 0.429 mm, an outer diameter of 0.789 mm, a wall thickness of 0.180 for a wall thickness-to-ID ratio of 0.42. The parison was stretched axially 3X, was stretched 7X ID and 3.8X OD to form a 3.0 mm balloon having a wall thickness of 0.0136mm. The balloon was heat set. The balloon had a burst pressure of 24 bars (23.7 atm) and a calculated radial tensile strength of about 38,900 psi. This balloon corresponds to the balloon designated "F" in Table 1.

## Example 2

A parison was formed in the manner described above in Example 1 to have an inner diameter of 0.429 mm and an outer diameter of 0.638 mm, having a parison wall thickness/ID ratio of 0.25. The parison was stretched axially 3.3X, was stretched 7X ID and 4.7X OD to produce a heat set 3.0 mm balloon having a wall thickness of .0054 mm measured burst strength of 15 bars (14.8 atm) and a calculated radial tensile strength of about 61,250 psi. This balloon corresponds to balloon G in Table 1.

## Example 3

A parison was formed in the manner described above in Example 1 to have an ID of 2.86 mm and an OD of 3.39 mm, with a wall thickness/ID ratio of 0.09. The parison was stretched at 90°C in an axial direction 3.75X and radially 7X ID and 5.9X OD to produce a heat set 20 mm balloon having a wall thickness of .00806, a measured burst strength of 5 bars (4.9 atm) and calculated radial tensile strength of 91,200 psi. This balloon corresponds to balloon H in Table 1.

It should be understood that the foregoing description of the invention is intended merely to be illustrative and that other embodiments and modifications may be apparent to those skilled in the art without departing from its spirit.

For example, the preferred aromatic linear polyester described for making balloons in accordance with th invention is polyethylene terephthalate derived from an aromatic dicarboxylic acid or its derivatives as a main acid component and an aliphatic glycol as a main glycol component. This polyester is a melt extrudable orientable semicrystalline polymer that can be fabricated into a variety of formed structures. Typical examples of other aromatic dicarboxylic acid polymers that meet these criteria utilize materials such as terepthalic acid, isothalic acid, napthalene dicarboxylic acid, together with aliphatic polymethylene glycols having two to ten carbon atoms. Among these are ethylene glycol, trimethylene glycol, tetramethylene glycol, pentamethylene glycol, hexamethylene glycol, didecamethylene glycol and cyclohexane dimethanol.

Having thus described the invention, what I desire to claim and secure by letters patent is:

## Claims

1. A biaxially oriented, flexible, polymeric balloon having a wall thickness to diameter ratio of less than $5.0 \times 10^{-3}$ and a radial tensile strength greater than about 35,000 psi.

2. A balloon as defined in claim 1 wherein the radial tensile strength is between 35,000 and 90,000 psi.

3. A balloon as defined in claim 1 wherein said balloon has a burst strength of up to about 25 bars.

4. A biaxially oriented, semicrystalline, flexible polymeric balloon having a radial tensile strength greater than about 35,000 psi.

5. A balloon as defined in claim 4 having a burst strength of up to about 25 bars.

6. A balloon as defined in any claims 1-5, said balloon being heat set.

7. A balloon as defined in any of claims 1-6 wherein the polymer comprises polyethylene terephthalate.

8. A dilatation catheter having a shaft portion and a balloon portion, the balloon comprising the balloon of any of claims 1-7.

9. Biaxially oriented, flexible, polymeric balloon heat set having an F5 radial tensile strength greater than 30,000 psi.

10. A method for forming a biaxially oriented flexible polymeric balloon comprising providing a thin walled tubular parison of orientable semicrystalline polymer having a wall to inner diameter ratio of less than 0.45; at a temperature within the range extending from the second order transition temperature to the first order transition temperature, drawing said parison axially to a length which is 2.5 to 6 times the starting length thereof and thereafter expanding the drawn tubing radially to an internal diameter which is six to eight times the initial internal diameter of the parison into an outer diameter which is equal to or greater than about four times the starting outer diameter of the parison, and while maintaining the balloon in said expanded state, raising the temperature of the balloon above the stretching temperature to raise the crystalline content of the balloon material, followed by cooling the drawn, expanded and heat set tubing to temperature less than its second order transition temperature.

11. A method as defined in claim 10 wherein the expanding means is pressurized fluid applied to the inside of the tubing.

12. A method of claim 10 wherein the polymer comprises polyethylene terephthalate.

*Fig. 1*

Fig. 2